# EUROPÄISCHE PATENTSCHRIFT

(11) **EP 3 114 465 B1**
(45) Veröffentlichungstag und Bekanntmachung des Hinweises auf die Patenterteilung: **28.03.2018**
(21) Anmeldenummer: 15703788.8
(22) Anmeldetag: 12.02.2015
(51) Int. Cl.: G01N 25/16, G01N 33/44

(54) **VERFAHREN UND VORRICHTUNG ZUR FESTSTELLUNG VON SCHRUMPFCHARAKTERISTIKA EINER FLÄCHIGEN MATERIALBAHN AUS THERMOPLASTISCHEM KUNSTSTOFF**
METHOD AND DEVICE FOR ASCERTAINING SHRINKAGE CHARACTERISTICS OF A FLAT MATERIAL STRIP MADE OF A THERMOPLASTIC MATERIAL
PROCÉDÉ ET DISPOSITIF DE DÉTERMINATION DE CARACTÉRISTIQUES DE RÉTRACTION D'UNE BANDE PLATE DE MATIÈRE THERMOPLASTIQUE

(30) Priorität: 05.03.2014 DE 102014102911
(43) Veröffentlichungstag der Anmeldung: 11.01.2017
(73) Patentinhaber: Krones AG, 93073 Neutraubling (DE)
(72) Erfinder: NAPRAVNIK, Christian, 93073 Neutraubling (DE)
(74) Vertreter: Benninger, Johannes
(86) Internationale Anmeldenummer: PCT/EP2015/052941
(87) Internationale Veröffentlichungsnummer: WO 2015/132057

(56) Entgegenhaltungen:
- CN-A- 101 957 332
- CN-A- 102 507 635
- JP-A- 2001 183 318
- US-A- 6 007 240
- US-A1- 2013 030 723
- TONG H M: "THICKNESS-DIRECTION COEFFICIENT OF THERMAL EXPANSION MEASUREMENT OF THIN POLYMER FILMS", REVIEW OF SCIENTIFIC INSTRUMENTS, AIP, MELVILLE, NY, US, Bd. 62, Nr. 2, Februar 1991 (1991-02), Seiten 422-430, XP000219378, ISSN: 0034-6748, DOI: 10.1063/1.1142137

## Beschreibung

Die vorliegende Erfindung betrifft ein Verfahren sowie eine Vorrichtung zur Feststellung von Schrumpfcharakteristika einer flächigen Materialbahn aus thermoplastischem Kunststoff.

Flächige Materialbahnen aus thermoplastischem Kunststoff finden beispielsweise als Folie für Schrumpfprozesse in der Packtechnik Verwendung. Zur Herstellung der flächigen Materialbahn werden die Kunststoffe üblicherweise als Granulat oder in Pulverform angeliefert, einem Extruder zugeführt und anschließend als thermoplastischer Kunststoff über eine Breitschlitzdüse ausgegeben.

Die jeweilige thermoplastische Flächenbahn bzw. die jeweilige Schrumpffolie wird vor dem Aufschrumpfen als Endlosmaterial auf Rollen bereit gestellt, anschließend an mehreren Artikeln angeordnet, nachfolgend zusammen mit den jeweiligen Artikeln durch einen Schrumpftunnel geführt und dort zur Schrumpfung mit Temperatur beaufschlagt. Treten die Artikel aus dem Schrumpftunnel aus, so ist eine als Verpackungseinheit ausgebildete Zusammenstellung aus Artikeln gebildet, bei welcher vermittels der aufgeschrumpften thermoplastischen Flächenbahn bzw. der Schrumpffolie die einzelnen Artikel zusammengehalten werden.

Eine derartige Vorrichtung zeigt beispielsweise die DE 10 2010 032 455 A1. Der Schrumpftunnel, welcher in der DE-Patentanmeldung offenbart wird, besitzt mehrere entlang einer Transportstrecke der Artikel angeordnete Heizstrahler, welche gemeinsam an einer Trägerkonstruktion fixiert und zusammen mit der Trägerkonstruktion zur Erwärmung der Artikel heb- und senkbar ausgebildet sind. Wird die jeweilige Schrumpffolie durch die Heizstrahler mit Temperatur beaufschlagt, kann sie sich zu Beginn ggf. um einen kleinen Betrag bis zu ihrem kristallinen Schmelzpunkt ausdehnen, um danach auf einen bestimmten Wert zu schrumpfen.

Um das Schrumpfergebnis zu optimieren, können die einzelnen Heizstrahler gemäß Vorrichtung der DE-Patentanmeldung gezielt angesteuert werden. Die jeweilige Temperierung muss hierbei in Abhängigkeit von Materialeigenschaften der Schrumpffolie erfolgen, damit diese zum Zusammenhalt der Artikel definiert auf die jeweiligen Artikel aufgeschrumpft werden kann. Derartige Materialbahnen aus thermoplastischem Kunststoff bzw. Schrumpffolien sind aus Kettenmolekülen aufgebaut, deren Zusammenhalt über zwischenmolekulare Bindungskräfte hergestellt wird. In Abhängigkeit der Makromoleküle sind die Kräfte unterschiedlich stark ausgebildet und beeinflussen somit das thermische, mechanische und chemische Verhalten der jeweiligen flächigen Materialbahn. Eine genaue Kenntnis der Materialcharakteristik der jeweiligen thermoplastischen Flächenbahn bzw. der jeweiligen Schrumpffolie ist daher unabdingbar, um einen Schrumpfprozess optimiert ausgestalten zu können.

Ist die Materialcharakteristik nicht oder unzureichend bekannt, so können die über die jeweilige Schrumpffolie nach Schrumpfung zusammengehaltenen Artikel ggf. eine instabile Lagerung besitzen. Ebenso können mit suboptimaler Wahl des jeweiligen Schrumpfmaterials ein Reißen des Materials und/oder ein Fließen des Materials bei Schrumpfung einhergehen.

Auch können in der Praxis thermoplastische Folien mit Kennzeichnungen, wie Produktbezeichnungen, verschiedenen Grafiken oder dergleichen versehen sein. Wünschenswert sind Schrumpfprozesse, bei welchen nach Aufschrumpfen der jeweiligen Folie auf die Artikel eine Proportionalität der Kennzeichnungen zumindest weitgehend beibehalten werden kann. Eine Über- oder unterproportionale Dehnung, beispielsweise in Längsrichtung, soll hierbei zumindest weitgehend vermieden werden.

Ein weiteres Problem, welches häufig bei aus dem Stand der Technik bekannten Schrumpfprozessen auftritt, ist eine nicht erwünschte Faltenausbildung der jeweiligen Folie nach Aufschrumpfung auf die Artikel. Da der ästhetische Gesamteindruck der jeweiligen Gebinde mit Schrumpffolie durch derartige Falten beeinflusst wird, sind auch aus diesem Grunde verbesserte Vorrichtungen und Verfahren wünschenswert, um vor Aufschrumpfung von thermoplastischen Materialien auf Artikel präzise Aussagen zum Verhalten des jeweiligen Materials bei Wärmeeinwirkung treffen zu können.

Die präzise Ermittlung von Materialcharakteristika ist durch Komplexität gekennzeichnet. Um Aussagen über das Verhalten der jeweiligen thermoplastischen Flächenbahn bei Aufschrumpfung treffen zu können, sind aus dem Stand der Technik bereits Vorrichtungen und Verfahren bekannt.

Beispielsweise besteht die Möglichkeit Materialeigenschaften über ein Probenstück der jeweiligen thermoplastischen Flächenbahn im Zugversuch zu ermitteln. Hierbei wird ein Probenstück über zwei relativ zueinander bewegbare Klemmbacken gehalten und anschließend bis zum Bruch bei einer relativen Bewegung der beiden Klemmbacken gedehnt. Eigenschaften des Materials werden während der Dehnung bis zum Bruch rechnergestützt erfasst und dienen als Information über die jeweiligen mechanischen Eigenschaften des Probenstücks und seines zugehörigen Materials. Bei derartigen Vorrichtungen kann das Verhalten des Materials lediglich in eine Ausdehnungsrichtung bzw. bei Krafteinwirkung in einer Richtung erfasst werden. Zudem besitzen aus dem Stand der Technik bekannte Einrichtungen zur Ermittlung der Materialeigenschaften im Zugsversuch eine große Dimensionierung und hohe Anschaffungskosten. Soll die Materialcharakteristik von thermoplastischen Flächenbahnen genau ermittelt werden, so sind mehrere Zugversuche vorzunehmen, woraus zudem ein hoher Zeitaufwand resultiert.

Auch sind Verfahren aus dem Stand der Technik bekannt, bei welchen ein Probenstück des jeweiligen thermoplastischen Materials in temperiertes Öl mit vorher festgelegter Temperatur vollständig eingesetzt wird. Nach einer spezifizierten Verweilzeit im Öl wird die Schrumpfung des Materials erfasst, wobei Rückschlüsse zur jeweiligen Materialeigenschaft gezogen werden. Betrachtend einen dynamischen Schrumpfungsprozess, bei welchem die jeweilige Schrumpffolie bzw. das jeweilige thermoplastische Material über einen Zeitverlauf mit sich ggf. ändernden Temperaturbedingungen beaufschlagt wird, sind die aus den aus dem Stand der Technik gewonnen Informationen zu den Materialeigenschaften häufig nicht ausreichend, um genaue Vorhersagen über das Verhalten des Materials bei Schrumpfung treffen zu können.

Durch die US 6,007,240 A werden weiterhin ein Verfahren und eine Vorrichtung zur thermomechanischen Analyse beschrieben. Die Vorrichtung zur thermomechanischen Analyse umfasst eine Temperiereinrichtung, ein Mittel zum Ausüben einer Kraft auf eine Probe, beispielsweise in Form eines Gewichtes, einen Positionssensor, eine Datenstation, eine Gewichtskontrolleinrichtung, Klemmen, zwischen welchen eine Probe eingeklemmt werden kann, und einen Temperatursensor. Eine Probe kann an den Klemmen befestigt und im Bereich der Temperiereinrichtung bzw. mittels der Temperiereinrichtung erwärmt werden. Die Längenänderung kann dabei durch den Positionssensor gemessen und der Temperaturänderung über den Zeitverlauf gegenübergestellt werden.

Aus dem Dokument CN 102507635 A ist ein Verfahren zur Messung der Wärmeschrumpfungsrate eines keramischen Faserprodukts bekannt. Dazu wird eine Probe mit mehreren Markierungen versehen. Anschließend kann diese auf eine Spannvorrichtung gelegt und über einem bestimmten Zeitraum erwärmt werden. Über eine Digitalkamera werden Fotos vom Umfang der Markierungen gemacht und auf einem Computer übermittelt. Der Computer berechnet mit Hilfe eines CAD Programms die praktische Länge des gemessenen Markers entsprechend der proportionalen Beziehung. Als nächster Schritt kann die Messung der Länge der Markierungspunkte vor dem Erhitzen und nach dem Erhitzen folgen, um die Schrumpfungsrate zu berechnen zu können.

Aufgabe der vorliegenden Erfindung ist daher ein Verfahren sowie eine Vorrichtung zur Verfügung zu stellen, mittels welchen genauere Aussagen zum Verhalten eines flächigen Bahnmaterials aus thermoplastischem Kunststoff bei Schrumpfung getroffen werden können. Zudem soll das Verfahren einfach durchführbar sein sowie die Vorrichtung einen unkomplizierten Aufbau besitzen.

Die obigen Aufgaben werden durch ein Verfahren sowie eine Vorrichtung gelöst, die die Merkmale in den Patentansprüchen 1 und 9 umfassen. Weitere vorteilhafte Ausgestaltungen werden durch die Unteransprüche beschrieben.

Die Erfindung betrifft ein Verfahren zur Feststellung von Schrumpfcharakteristika einer flächigen Materialbahn aus thermoplastischem Kunststoff. Insbesondere kann in diversen Ausführungsformen bei Feststellung der Schrumpfcharakteristika ein Längs- und/oder Querschrumpf der jeweiligen flächigen Materialbahn aus thermoplastischem Kunststoff festgestellt werden.

Im Rahmen des erfindungsgemäßen Verfahrens wird ein Probenstück definierter Abmessung einer Erwärmung oberhalb einer für das jeweilige Material des Probenstücks wirksamen Schrumpfungstemperatur unterzogen. Das Probenstück kann beispielsweise quaderförmig bzw. quadratisch ausgebildet sein und besitzt eine Dimensionierung, welche eine nachfolgend noch näher beschriebene und aus einer Erwärmung resultierende Dimensionsänderung problemlos erkennen lassen. Dem angesprochenen Fachmann ist klar, dass er für die jeweilige Abmessung des Probenstücks zahlreiche geeignete Geometrien verwenden kann, so dass die Erfindung nicht auf quaderförmige bzw. quadratische Probenstücke beschränkt ist.

Beispielsweise ist vorstellbar, dass das jeweilige Probenstück durch Beaufschlagung über ein gasförmiges Medium, wie Heißluft oder dergleichen, erwärmt wird. In bevorzugten Ausführungsformen kann es jedoch sein, dass das jeweilige Probenstück unter Wirkverbindung mit einer Heizplatte erwärmt wird und auf der Heizplatte - ggf. mit einem der Heizplatte und dem jeweiligen Probenstück zwischengeordnetem Trägersubstrat - flächig aufliegt. Vorteilhafterweise kann bei Erwärmung des Probenstücks durch eine Heizplatte eine zumindest weitgehend gleichmäßige Erwärmung des gesamten Probenstücks erfolgen.

Denkbar ist beispielsweise, dass die Heizplatte zumindest annäherungsweise Umgebungstemperatur besitzt, das Probenstück auf der Heizplatte bzw. auf dem ggf. zwischengeordnetem Trägersubstrat angeordnet wird und hierauf folgend eine Inbetriebnahme der Heizplatte mit zunehmender Erwärmung des Probenstücks erfolgt. In weiteren Ausführungsformen ist ebenso vorstellbar, dass die Heizplatte bis zu einer gewissen Temperatur oder einem gewissen Temperaturniveau vorerwärmt und hierauf folgend das jeweilige Probenstück auf der Heizplatte - bzw. ggf. auf dem der Heizplatte und dem Probenstück zwischengeordneten Trägersubstrat - angeordnet wird.

In bevorzugten Ausführungsformen wird das Probenstück in einer Aufnahme angeordnet. Die Aufnahme kann flächig zur Erwärmung des Probenstücks mit der Heizplatte in Kontakt gebracht sein. Weiter kann das Trägersubstrat, auf welches das Probenstück ggf. aufgelegt wird, bei Erwärmung sowie bei Erfassen der jeweiligen Dimensionsänderung in der Aufnahme angeordnet sein. Der definitionsgemäße Terminus "Aufnahme" der nachfolgend noch näher beschriebenen Vorrichtung kann somit als physischer Bestandteil der nachfolgend noch näher beschriebenen Vorrichtung verstanden werden. Auch kann der Begriff "Aufnahme" in weiteren Ausführungsformen als Bereich definiert sein, innerhalb dessen das jeweilige Probenstück bei Erwärmung angeordnet ist.

Die Heizplatte kann derart ausgebildet sein, dass sie bis zu einer gewissen Temperatur bzw. einem gewissen Temperaturniveau erwärmt wird und anschließend die gewisse Temperatur bzw. das gewisse Temperaturniveau beibehält. Beispielsweise kann die Temperatur auf 130°C oder 150°C festgesetzt sein. Der Fachmann kann die entsprechende Temperatur unter Berücksichtigung eines vorgesehen späteren Schrumpfungsprozesses, bei dem das jeweilige thermoplastische Material auf Artikel aufgeschrumpft wird, wählen.

Weiter wird nach Erwärmung über den Zeitverlauf eine Dimensionsänderung des Probenstücks im voranschreitenden Schrumpfungsprozess beobachtet und/oder gemessen. Das Beobachten der voranschreitenden Schrumpfung kann hierbei zu einem bestimmten Zeitpunkt nach Erwärmen des Probenstücks beginnen und an einem bestimmten Zeitpunkt bzw. nach bestimmter Dauer enden. Beispielsweise kann vorgesehen sein, dass das Beobachten und/oder Messen der Dimensionsänderung des Probenstücks direkt nach Erreichen einer für das jeweilige Material wirksamen Schrumpfungstemperatur begonnen wird. Derartige Ausführungsformen haben sich insbesondere bewährt, da die Dimensionsänderung direkt nach Erreichen das für das jeweilige Material wirksamen Schrumpfungstemperatur beginnt und somit ggf. die gesamte Dimensionsänderung des jeweiligen Probenstücks über den Zeitverlauf erfassbar ist.

Hat das Probenstück eine vorbestimmte Temperatur erreicht, so kann im Rahmen diverser Ausführungsformen eine nachfolgend noch näher beschriebene Temperiereinrichtung derart gesteuert werden, dass die Temperatur des Probenstücks während Beobachten und/oder Messen der Dimensionsänderung zumindest weitgehend beibehalten wird.

In weiteren Ausführungsformen der vorliegenden Erfindung kann das Temperaturniveau des Probenstücks über den Zeitverlauf und während Beobachten und/oder Messen der Dimensionsänderung variiert werden. Bevorzugt können hierbei verschiedene Temperaturwerte vorgegeben sein, auf welcher das Probenstück über den Zeitverlauf und während des Erfassens und/oder Beobachtens der Dimensionsänderung temperiert wird. Unter Berücksichtigung eines Schrumpfungsprozesses, bei welchem das jeweilige thermoplastische Material auf Artikel aufgeschrumpft ist, kann es sein, dass die jeweilige Temperatur bei Schrumpfung nicht durchgehend konstant ausgebildet ist. Vermittels einer Variation der Temperatur über den Zeitverlauf bei erfindungsgemäßer Vorrichtung bzw. erfindungsgemäßem Verfahren können somit präzises Aussagen gewonnen werden, wie sich das jeweilige thermoplastische Material bei sich über den Zeitverlauf ändernder Temperierung verhält.

Zudem wird im Rahmen des erfindungsgemäßen Verfahrens ein Zusammenhang zwischen der beobachteten und/oder gemessenen Dimensionsänderung des Probenstücks, der Dauer des Schrumpfungsprozesses sowie der jeweiligen Temperatur und/oder Temperaturänderung des Probenstücks im Schrumpfungsprozess hergestellt.

In bevorzugten Ausführungsformen wird die jeweilige Temperatur und/oder Temperaturänderung des Probenstücks zumindest während des Beobachtens und/oder Messens der Dimensionsänderung durchgehend erfasst. Zwischen der durchgehend erfassten Temperatur und/oder Temperaturänderung kann weiterhin ein Zusammenhang mit der Dimensionsänderung sowie der Dauer des jeweiligen Schrumpfungsprozesses hergestellt werden.

Vorzugsweise wird die Temperatur und/oder die Temperaturänderung des Probenstücks über einen oder mehrere Infrarotsensoren erfasst. Die ein oder mehreren Infrarotsensoren können hierbei oberhalb des Probenstücks angeordnet sein, wobei sich das jeweilige Probenstück im Erfassungsbereich der ein oder mehreren Infrarotsensoren befindet. Vorteilhafterweise ist bei Verwendung von einem oder mehreren Infrarotsensoren kein direkter Kontakt von Sensoren zum Probenstück notwendig, so dass die Dimensionsänderung des Probenstücks während des voranschreitenden Schrumpfungsprozesses unverfälscht erfasst und durch einen Kontakt mit Sensoren nicht beeinflusst wird.

Auch kann es sein, dass das Probenstück auf ein Trägersubstrat aufgelegt wird, welches zur Erwärmung thermische Energie an das jeweilige Probenstück weitergibt bzw. thermische Energie an das jeweilige Probenstück überträgt. Bei Auflegen des Probenstücks auf das jeweilige Trägersubstrat kann das Probenstück über eine seiner Breitseitenflächen mit dem Trägersubstrat in Oberflächenkontakt stehen, während die weitere gegenüberliegende Breitseitenfläche des Probenstücks keinen Oberflächenkontakt zum Trägersubstrat besitzt. Die weitere gegenüberliegende Breitseitenfläche kann hierbei ggf. den ein oder mehreren Infrarotsensoren und/oder ein oder mehreren weiteren Sensoren zugewandt sein. In bevorzugten Ausführungsformen steht eine Breitseitenfläche des jeweiligen Probenstücks vollständig mit dem Trägersubstrat in Oberflächenkontakt.

Das Trägersubstrat kann in bevorzugten Ausführungsformen durch ein flüssiges Medium, wie Öl oder dergleichen, ausgebildet sein. Die Viskosität des Trägersubstrates bzw. des flüssigen Mediums ist hierbei bevorzugt derart zu wählen, dass das Probenstück über das Trägersubstrat getragen werden kann, ohne dass ein Oberflächenkontakt des Probenstücks zu einer unterhalb des Trägersubstrates ggf. angeordneten Heizplatte und/oder einer unterhalb des Trägersubstrates ggf. angeordneten Aufnahme für das Probenstück ausgebildet ist. Ein Anhaften des jeweiligen Probenstücks an der Heizplatte bzw. an der Aufnahme wird hierdurch vermieden.

Demnach kann das jeweilige Trägersubstrat beispielsweise als Ölfilm ausgebildet sein. Das jeweilige Probenstück kann hierbei nach Auflegen auf das jeweilige Trägersubstrat schwimmend vom jeweiligen Trägersubstrat getragen werden, jedoch hierbei nicht vollständig in das Trägersubstrat eintauchen. Erfährt das Probenstück während eines voranschreitenden Schrumpfprozesses eine Dimensionsänderung, so kann sich hierbei das jeweilige Probenstück aufgrund der schwimmenden Lagerung auf dem Trägersubstrat zumindest weitgehend ohne Reibungswiderstände bewegen. Eine sehr genaue Erfassung der Schrumpfcharakteristika ohne Verfälschung von Messergebnissen ist bei Ausbildung des Trägersubstrates über ein flüssiges und vorzugsweise durch Öl ausgebildetes Medium hierbei möglich. Auch besteht die Möglichkeit das Trägersubstrat durch weitere Materialien bzw. Stoffe, wie beispielsweise Talkum, auszubilden. Das jeweilige Trägersubstrat kann hierbei über die Temperiereinrichtung bzw. ggf. über eine Heizplatte erwärmt werden und thermische Energie an das Probenstück weitergeben.

In weiteren Ausführungsformen ist vorstellbar, dass das Probenstück vor Erwärmung bereichsweise mit einem formstabilen Material flächig in Verbindung gebracht wird, welches formstabile Material derart ausgebildet ist, dass es bei Erwärmung des Probenstücks keine oder im Wesentlichen keine Dimensionsänderung erfährt. Beispielsweise kann das formstabile Material auf das Probenstück aufgeklebt werden. Vorzugsweise kann das formstabile Material auf wenigstens eine Breitseitenfläche des Probenstücks aufgeklebt werden. Insbesondere kann das formstabile Material auf die wenigstens eine Breitseitenfläche derart aufgeklebt werden, dass es sich quer über die gesamte Breitseitenfläche erstreckt. Vorstellbar ist beispielsweise, dass das formstabile Material als Streifen bzw. streifenförmig ausgebildet ist.

In diversen Ausführungsformen kann hierbei vorgesehen sein, dass das jeweilige Probenstück mit mehreren Einheiten aus formstabilen Materialien bzw. mit mehreren ggf. streifenförmig ausgebildeten Einheiten aus formstabilen Materialien in Verbindung gebracht wird. Beispielsweise können die Einheiten aus formstabilen Materialien in Randbereichen des jeweiligen Probenstücks angeordnet sein. Weitere Ausführungsformen sind vorstellbar, bei welchen die ggf. streifenförmig ausgebildeten formstabilen Materialien beispielweise zueinander lotrecht orientiert und/oder in weiteren Orientierungen auf das jeweilige Probenstück aufgebracht sind.

Da das formstabile Material einen Widerstand für das thermoplastische Material des Probenstücks bei Schrumpfung darstellt, können hiermit durch Behälter auftretende Widerstände für thermoplastische Folien während eines Schrumpfungsprozesses simuliert werden. Präzise Aussagen zum Verhalten des thermoplastischen Materials bzw. der Schrumpffolie bei Aufschrumpfen auf Artikel sind mittels der Verbindung des Probenstücks mit formstabilem Material möglich.

In weiteren Ausführungsformen ist vorstellbar, dass die zwei Einheiten aus formstabilem Material auf unterschiedliche Bereiche des Probenstücks aufgebracht werden, wobei die zwei Einheiten bei Dimensionsänderung des Probenstücks ortsfest gehalten werden. Beispielweise können die zwei Einheiten aus formstabilen Materialien in einer Aufnahme der nachfolgend noch näher beschriebenen Vorrichtung geklemmt werden. Erfährt das Probenstück aus der Erwärmung resultierend einen Schrumpfungsprozess, so wird das Probenstück aufgrund der ortsfesten Anordnung der zwei Einheiten aus formstabilen Materialien ggf. bis zum Bruch gedehnt. Unter Berücksichtigung der Dimensionsänderung bei Dehnung und dem ggf. resultierendem Bruch können weitere Aussagen zum Verhalten des jeweiligen thermoplastischen Materials bei Schrumpfung gewonnen werden. Weitere Ausführungsformen, bei welchen mehr als zwei Einheit aus formstabilem Material auf das jeweilige Probenstück aufgebracht werden, sind ebenso vorstellbar.

Weiter kann es sein, dass ein erstes Probenstück des thermoplastischen Kunststoffes vor Erwärmung bereichsweise mit dem formstabilen Material in Verbindung gebracht wird und ein zweites Probenstücks des thermoplastischen Kunststoffes nicht mit dem formstabilen Material in Verbindung gebracht wird. Das erste und das zweite Probenstück können zeitgleich erwärmt werden und ein Zusammenhang unter Berücksichtigung der Dimensionsänderungen des ersten und des zweiten Probenstücks im voranschreitenden Schrumpfungsprozess, der jeweiligen Temperatur und/oder Temperaturänderung des ersten und des zweiten Probenstücks im Schrumpfungsprozess sowie der Dauer des Schrumpfungsprozesses hergestellt werden. Detailliertere Aussagen zum Verhalten des jeweiligen thermoplastischen Materials bei Aufschrumpfung auf Artikel lassen sich mittels dieser Ausführungsform zeitoptimiert treffen.

Erfindungsgemäß ist vorgesehen, dass die Dimensionsänderung des Probenstücks im voranschreitenden Schrumpfungsprozess vermittels optischer Erkennung über ein oder mehrere Kamerasysteme beobachtet wird. Die Kamerasysteme können oberhalb des Probenstücks und beispielsweise benachbart bzw. im Bereich eines Infrarotsensors zur Ermittlung der Temperatur und/oder Temperaturänderung des Probenstücks angeordnet sein. Um eine möglichst genaue Erkennung der jeweiligen Dimensionsänderung des Probenstücks im voranschreitenden Schrumpfungsprozess zu gewährleisten, haben sich hierbei die vorherig beschriebenen Ausführungsformen bewährt, bei welchen das jeweilige Probenstück auf ein durch Öl oder Talkum ausgebildetes Substrat aufgelegt und durch das Substrat tragend gehalten wird. Die dem Substrat abgewandte Breitseitenfläche des Probenstücks befindet sich hierbei im Erfassungsbereich der ein oder mehreren Kamerasysteme, so dass eine Dimensionsänderung des jeweiligen Probenstücks über den Zeitverlauf durch die ein oder mehreren Kamerasysteme vollständig erfassbar ist. Da das Probenstück ggf. schwimmend auf das Trägersubstrat aufgelegt ist und eine dem Kamerasystem bzw. den ein oder mehreren Infrarotsensoren zugewandte Breitseitenfläche des jeweiligen Probenstücks bevorzugt nicht mit dem Trägersubstrat in Kontakt gebracht ist, kann bei derartiger Ausführung der vorliegenden Erfindung eine sehr genau Erfassung von Dimensionsänderungen des jeweiligen Probenstücks sowie der Temperatur und/oder Temperaturänderung des jeweiligen Probenstücks erfolgen.

Um die Erfassung der jeweiligen Dimensionsänderung weiter zu verbessern, kann vorgesehen sein, dass zur optischen Erkennung der Dimensionsänderung wenigstens eine visuelle Kennzeichnung auf das jeweilige Probenstück aufgebracht wird. Die optische Erkennung der Dimensionsänderung kann, ggf. durch die ein oder mehreren Kamerasysteme unter Berücksichtigung der visuellen Kennzeichnung erfolgen. Der Terminus "visuelle Kennzeichnung" ist im Rahmen vorliegender Erfindung breit auszulegen, so dass jegliche Oberflächen des Probenstücks eine definitionsgemäße "visuelle Kennzeichnung" besitzen können, bei welchen wenigstens eine Breitseitenfläche des Probenstücks bzw. die in Richtung des Kamerasystems weisende Breitseitenfläche des Probenstücks bereichsweise einen verminderten oder erhöhten Kontrast besitzt.

In einer einfachen Ausführungsform kann die visuelle Kennzeichnung beispielsweise als Linie ausgebildet sein, die sich über das jeweilige Probenstück erstreckt. In Abhängigkeit der sich aus dem voranschreitenden Schrumpfungsprozess des Probenstücks ändernden Linienform und/oder Linienorientierung und/oder Linienerstreckung können hierbei Rückschlüsse zur jeweiligen Dimensionsänderung des Probenstücks gezogen werden.

In weiteren Ausführungsform kann die visuelle Kennzeichnung über eine definierte Fläche auf das Probenstück aufgebracht sein, wobei die visuelle Kennzeichnung im Bereich der definierten Fläche eine gegenüber der Oberfläche des Probenstücks verminderten oder erhöhten Kontrast besitzt. Ausführungsformen, bei welchen die visuelle Kennzeichnung durch einen Schriftzug, ein Symbol der dergleichen ausgebildet ist, sind hierbei vorstellbar.

Die vorliegende Erfindung betrifft darüber hinaus eine Vorrichtung zur Feststellung von Schrumpfungscharakteristika einer flächigen Materialbahn aus thermoplastischem Kunststoff.

Die Vorrichtung umfasst eine Aufnahme für ein Probenstück des jeweiligen thermoplastischen Kunststoffs. Die Aufnahme kann von außen zugänglich sein. In bevorzugten Ausführungsformen kann das Probenstück in die Aufnahme der Vorrichtung eingesetzt bzw. eingelegt werden.

Weiter umfasst die Vorrichtung eine Temperiereinrichtung, welche mit der Aufnahme zur Erwärmung des Probenstücks oberhalb einer für das jeweilige Material des Probenstücks wirksamen Schrumpfungstemperatur in Verbindung steht. Beispielsweise kann die Temperiereinrichtung eine Heizplatte aufweisen, welche die Aufnahme umfasst und/oder mit welcher eine Aufnahme des Probenstücks zur Entgegennahme von thermischer Energie in Oberflächenkontakt gebracht ist. Auch ist vorstellbar, dass die Temperiereinrichtung Heizelemente, wie Heizdrähte oder dergleichen, umfasst.

Zudem umfasst die Vorrichtung eine Detektionseinrichtung zum Messen und/oder Beobachten einer voranschreitenden Dimensionsänderung des Probenstücks über den Zeitverlauf während eines aus der Erwärmung resultierenden Schrumpfungsprozesses. Die Detektionseinrichtung kann hierbei oberhalb des Aufnahmebereichs für das Probenstück angeordnet sein.

Auch sind ein oder mehrere Mittel zum Erfassen einer Temperatur und/oder Temperaturänderung des Probenstücks im Schrumpfungsprozess vorgesehen. Die ein oder mehreren Mittels zum Erfassen einer Temperatur und/oder Temperaturänderung des Probenstücks können wenigstens einen Temperatursensor umfassen, der mit dem jeweiligen Probenstück zur Erfassung seiner jeweiligen Ist-Temperatur in Verbindung gebracht ist.

Darüber hinaus umfasst die Vorrichtung eine rechnergestützte Einheit, die mit den ein oder mehreren Mittelns zum Erfassen einer Temperatur und/oder einer Temperaturänderung des Probenstücks sowie mit der wenigstens einen Detektionseinrichtung zum Messen und/oder Beobachten einer Dimensionsänderung des jeweiligen Probenstücks in Wirkverbindung gebracht ist.

Die rechnergestützte Einheit kann hierzu an die ein oder mehreren Mittel und/oder an die wenigstens eine Detektionseinrichtung zum Austausch von Daten kabellos oder via Kabelverbindung gekoppelt sein. Hierbei können Daten zur jeweiligen Temperatur und/oder einer jeweiligen Temperaturänderung des Probenstücks an die jeweilige rechnergestützte Einheit gesendet bzw. von der jeweiligen rechnergestützten Einheit empfangen werden. Auch können Daten zur jeweiligen Dimensionsänderung von der Detektionseinrichtung an die rechnergestützte Einheit gesendet bzw. von der jeweiligen rechnergestützten Einheit empfangen werden.

Weiter ist vorgesehen, dass über die rechnergestützte Einheit ein Zusammenhang aus einer Dauer des Schrumpfungsprozesses, der erfassten Temperatur und/oder Temperaturänderung des Probenstücks im Schrumpfungsprozess sowie der über den Zeitverlauf voranschreitenden Dimensionsänderung des Probenstücks herstellbar ist.

Zudem kann es sein, dass der über die Einheit hergestellte Zusammenhang als Schrumpfcharakteristika durch die Vorrichtung ausgegeben bzw. dargestellt wird. Die Vorrichtung kann hierzu mit ein oder mehreren Anzeigeeinheiten, wie Bildschirmen oder dergleichen, in Verbindung gebracht sein. Auch kann in diversen Ausführungsformen vorgesehen sein, dass die Vorrichtung mit einer Druckeinrichtung in Verbindung steht, über welche der Zusammenhang bzw. die Schrumpfcharakteristika auf Papier festgehalten und ausgegeben werden können.

In bevorzugten Ausführungsformen kann weiter vorgesehen sein, dass die Vorrichtung eine an die rechnergestützte Einheit gekoppelte Speichereinheit aufweist, auf welcher der jeweils hergestellte Zusammenhang bzw. die Schrumpfcharakteristika durch die rechnergestützte Einheit ablegbar sind. Hierbei kann vor Ablegen eine Zuordnung des jeweils hergestellten Zusammenhangs bzw. der jeweiligen Schrumpfcharakteristika zu dem jeweiligen Probenstück erfolgen. Ein späterer Zugriff auf die jeweiligen Schrumpfcharakteristika eines thermoplastischen Materials ist aufgrund der Zuordnung zum jeweiligen Probenstück möglich. Für den angesprochenen Fachmann ist klar, dass die Vorrichtung geeignete Schnittstellen aufweisen kann, mittels welcher der jeweils hergestellte Zusammenhang bzw. die Schrumpfcharakteristika auf ein externes Speichermedium duplizierbar sind.

Weiter kann es sein, dass über die rechnergestützte Einheit aus einer durchgehend während dem Messen und/oder Beobachten voranschreitenden Dimensionsänderung ausgebildeten Erfassung einer Temperatur und/oder Temperaturänderung des Probenstücks ein Zusammenhang herstellbar ist. Da ebenso ein Schrumpfungsprozess von thermoplastischen Folien, wie er in einem Schrumpftunnel auftreten kann, als ein dynamischer Prozess mit gleichbleibender oder sich über den Zeitverlauf ändernder Schrumpfungstemperatur ausgebildet ist, können vermittels der durchgehenden Erfassung einer Temperatur und/oder Temperaturänderung des Probenstücks präzise Aussagen hinsichtlich eines zu erwartenden Verhaltens der jeweiligen Folie bei Aufschrumpfung auf Artikel gezogen werden.

Auch kann vorgesehen sein, dass die rechnergestützte Einheit mit der Temperiereinrichtung zur Erwärmung des Probenstücks in Verbindung steht. Weiter kann vorgesehen sein, dass die Temperatur des Probenstücks während dem Messen und/oder Beobachten der voranschreitenden Dimensionsänderung zumindest weitgehend konstant gehalten wird oder während dem Messen und/oder Beobachten der voranschreitenden Dimensionsänderung definiert verändert wird. Sofern ein Erfassen einer Temperatur und/oder Temperaturänderung während dem Messen und/oder Beobachten der voranschreitenden Dimensionsänderung durchgehend erfolgt, kann über die rechnergestützte Einheit die Temperiereinrichtung in Abhängigkeit der jeweiligen erfassten Ist-Temperatur des Probenstücks zumindest annäherungsweise in Echtzeit geregelt werden.

Weiter kann vorgesehen sein, dass die rechnergestützte Einheit nach Erwärmung des Probenstücks bei einer festgesetzten Temperatur des Probenstücks mit dem Messen und/oder Beobachten der jeweiligen Dimensionsänderung beginnt. Beispielsweise kann die festgesetzte Temperatur als eine für das jeweilige Probenstück wirksame Schrumpfungstemperatur ausgebildet sein.

In weiteren Ausführungsformen kann zudem vorgesehen sein, dass die ein oder mehreren Mittel zum Messen und/oder Erfassen einer Temperatur und/oder Temperaturänderung des Probenstücks wenigstens einen Infrarotsensor umfassen. Der Infrarotsensor kann oberhalb des Probenstücks beziehungsweise oberhalb der Aufnahme für das jeweilige Probenstück angeordnet sein und drahtlos oder drahtgebunden mit der rechnergestützten Einheit zum Übertragen von Daten der jeweiligen Temperatur und/oder Temperaturänderung des Probenstücks in Verbindung stehen.

Wie vorhergehend bereits erwähnt, umfasst die wenigstens eine Detektionseinrichtung zum Erfassen einer Dimensionsänderung des Probenstücks zumindest ein Kamerasystem. Ebenso kann das Kamerasystem oberhalb der Aufnahme für das jeweilige Probenstück angeordnet und drahtlos oder drahtgebunden mit der rechnergestützten Einheit zum Übertragen von Daten hinsichtlich der Dimensionsänderung des jeweiligen Probenstücks in Verbindung stehen.

Weiter kann das Probenstücks in der Aufnahme auf ein Trägersubstrat auflegbar sein, welches zu Weitergabe von thermischer Energie an das jeweilige Proebenstück mit einer Heizplatte der Temperiereinrichtung in Verbindung steht. Das Trägersubstrat kann somit auf die Heizplatte flächig aufgebracht sein.

In bevorzugten Ausführungsformen ist das Trägersubstrat durch Öl und/oder Talkum ausgebildet.

Insbesondere haben sich Ausführungsformen bewährt, bei welchen die rechnergestützte Einheit zur Weitergabe von Informationen über den hergestellten Zusammenhang mit einer Steuerungseinrichtung eines Schrumpftunnels in Verbindung steht. Eine Verbindung zwischen der rechnergestützten Einheit und der Steuerungseinrichtung des Schrumpftunnels kann mittels Kabelverbindung und/oder kabellos erfolgen. Vorstellbar ist hierbei beispielsweise, dass während einem Betrieb und/oder einem Betrieb des Schrumpftunnel vorausgehend ein bereits beschriebener Zusammenhang aus Dimensionsänderung des Probenstücks, Dauer des Schrumpfungsprozesses sowie Temperatur und/oder Temperaturänderung des Probenstücks hergestellt wird, die Steuerungseinrichtung des Schrumpftunnels über den hergestellten Zusammenhang informiert wird und eine Regelung des Schrumpftunnels unter Berücksichtigung der Information erfolgt.

Bevorzugt steht die Steuerungseinrichtung des Schrumpftunnels mit einer oder mehreren Einrichtung zur Temperaturbeaufschlagung von Artikeln, welche den Schrumpftunnel passieren, in Verbindung, wobei die ein oder mehreren Einrichtungen zur Temperaturbeaufschlagung über die Steuerungseinrichtung unter Berücksichtigung des hergestellten Zusammenhangs geregelt werden. Die Einrichtungen zur Temperaturbeaufschlagung können ein oder mehrere Heizeinrichtungen sowie ein oder mehrere Mittel zur Erzeugung eines Volumenstroms aufweisen.

In der Praxis haben sich zudem Ausführungsformen bewährt, bei welchen die Temperiereinrichtung der erfindungsgemäßen Vorrichtung durch eine elektrisch- und/oder gasbetriebene Heizung ausgebildet ist. Die Erfindung ist jedoch nicht auf die Verwendung von elektrischen- und/oder gasbetriebenen Heizungen beschränkt, so dass darüber hinaus auch alternative Einrichtungen zur Temperierung des Probenstücks Verwendung finden können.

Im Folgenden sollen Ausführungsbeispiele die Erfindung und ihre Vorteile anhand der beigefügten Figuren näher erläutern. Die Größenverhältnisse der einzelnen Elemente zueinander in den Figuren entsprechen nicht immer den realen Größenverhältnissen, da einige Formen vereinfacht und andere Formen zur besseren Veranschaulichung vergrößert im Verhältnis zu anderen Elementen dargestellt sind.
Figuren 1A und 1B zeigen schematische Ansichten von aus dem Stand der Technik bekannten Vorrichtungen zur Ermittlung von Schrumpfcharakteristika;
Figur 2 zeigt beispielhaft den Längsschrumpf sowie den Querschrumpf mehrere thermoplastischer Folien in Abhängigkeit der jeweiligen Schrumpfungstemperatur;
Figur 3 zeigt beispielhaft den Längsschrumpf sowie den Querschrumpf einer thermoplastischen Folie aus der Praxis;
Figur 4 zeigt eine schematische Ansicht eines Ausführungsbeispiels einer erfindungsgemäßen Vorrichtung zur Feststellung von Schrumpfungscharakteristika;
Figur 5 zeigt eine vergrößerte Ansicht des Ausschnitts A aus Figur 4;
Figur 6 zeigt eine schematische Draufsicht auf die Ausführungsform einer Vorrichtung aus Figur 4;
Figur 7 zeigt eine beispielhafte Ausführungsform eines Probenstücks vor und nach Erwärmung durch eine erfindungsgemäße Vorrichtung;
Figur 8 zeigt eine schematische Ansicht einer weiteren beispielhaften Ausführungsform eines Probenstücks;
Figur 9 zeigt eine schematische Ansicht einer weiteren beispielhaften Ausführungsform eines Probenstücks;
Figur 10 zeigt eine schematische Ansicht eines Probenstücks bei Umsetzung von möglichen Ausführungsformen eines erfindungsgemäßen Verfahrens;

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figur erforderlich sind. Die dargestellten Ausführungsformen stellen lediglich Beispiele dar, wie die erfindungsgemäße Vorrichtung oder das erfindungsgemäße Verfahren ausgestaltet sein können und stellen keine abschließende Begrenzung dar.

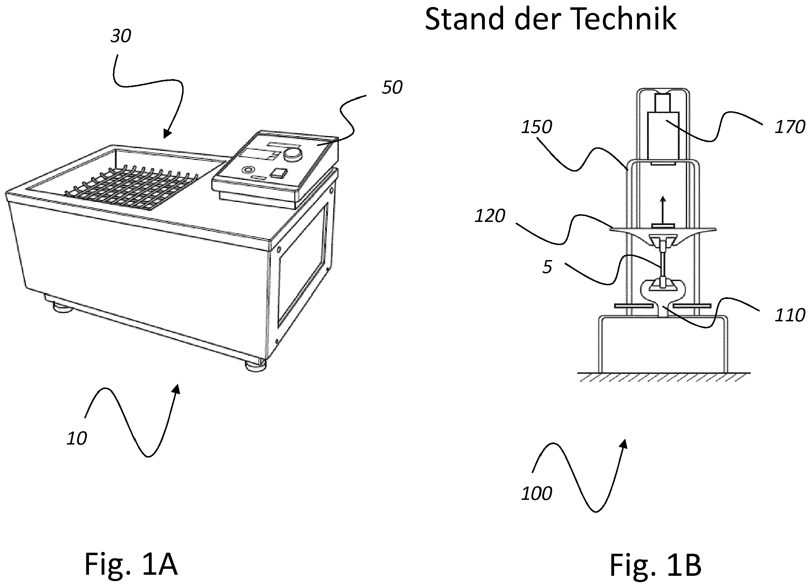

**Figuren 1** **A und 1B** zeigen schematische Ansichten von aus dem Stand der Technik bekannten Vorrichtungen zur Ermittlung von Schrumpfcharakteristika. Die in Figur 1A dargestellte Messvorrichtung 10 besitzt ein Flüssigkeitsbad 30, in welches Flüssigkeitsbad 30 ein thermoplastisches Material zur Feststellung von Schrumpfungscharakteristika vollständig eingelegt wird.

Das jeweilige im Flüssigkeitsbad 30 befindliche Medium, welches in der Praxis durch Öl ausgebildet sein kann, wird vor Einlegen des thermoplastischen Materials auf eine definierte Temperatur, beispielsweise 130°C oder 150°C, erwärmt. Die definierte Temperatur kann über den Temperaturregler 50 vorgegeben werden. Nachdem das thermoplastische Material bei erreichter definierter Temperatur des flüssigen Mediums in das Flüssigkeitsbad 30 eingelegt wurde, verweilt es dort über eine gewisse vorbestimmte Zeit und wird anschließend aus dem Flüssigkeitsbad entnommen.

Hierauf werden Dimensionsänderungen des thermoplastischen Materials erfasst und festgehalten. Das Verhalten bzw. die Dimensionsänderung des jeweiligen thermoplastischen Materials im Schrumpfungsprozess über den Zeitverlauf zu erfassen ist mittels der in Figur 1A gezeigten Messvorrichtung nicht möglich. Da weiter die Temperatur definiert festgesetzt wird, können mittels der in Figur 1A dargestellten Vorrichtungen keine Aussagen zu einem Verhalten es jeweiligen thermoplastischen Materials bei sich über den Zeitverlauf ändernder Temperierung bzw. bei Verhalten des Materials mit unterschiedlichen Temperaturen gewonnen werden.

Bei der in Figur 1B dargestellten Messvorrichtung 100 werden Schrumpfcharakteristika eines thermoplastischen Materials, durch welches das Probenstücks 5 ausgebildet ist, im Zugversuch ermittelt. Die Messvorrichtung 100 besitzt einen Maschinenrahmen 150, weiter einen unteren Spannkopf 110 sowie einen oberen Spannkopf 120, an welchen beiden Spannköpfen 110 und 120 das Probenstück 5 jeweils klemmend fixierbar ist.

Über den Arbeitskolben 170 ist der obere Spannkopf 120 relativ zum unteren Spannkopf 110 bewegbar. Die Bewegung ist in Figur 1B mittels Pfeildarstellung angedeutet. Bei relativer Bewegung des oberen Spannkopfes 120 zum unteren Spannkopf 110 wird das Probenstück 5 bis zum Bruch gedehnt. Der vom oberen Spannkopf 120 hierbei zurückgelegte Weg wird in Relation zur jeweiligen auf den oberen Spannkopf 120 durch das Probenstück 5 einwirkenden Kraft festgehalten und an eine rechnergestützte Einheit übertragen. Mit den gewonnenen Werten können Aussagen zum Verhalten des jeweiligen thermoplastischen Materials bei Schrumpfung gewonnen werden.

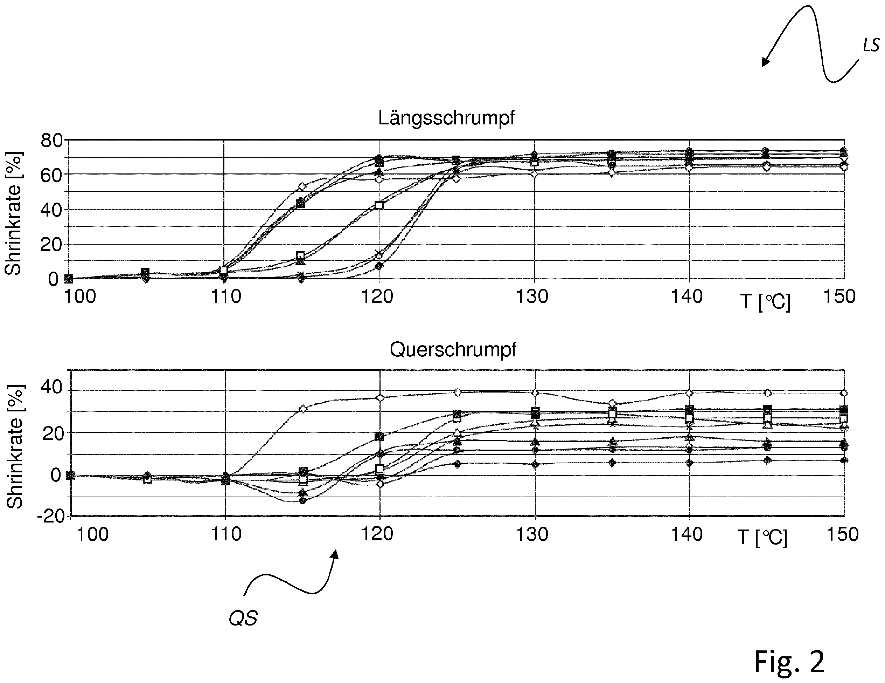

Bei der in Figur 1B dargestellten Vorrichtung muss das Probenstück 5 klemmend zwischen dem unteren Spannkopf 110 und dem oberen Spannkopf 120 fixiert werden. Aufgrund eines hiermit einhergehenden Zeitaufwandes sind in der Praxis Vorrichtungen wünschenswert, bei welchen auf derartige Fixierungen verzichtet werden kann. Weiter können mittels der in Figur 1B beispielhaft dargestellten Messvorrichtung 100 lediglich Aussagen zum Verhalten des jeweiligen thermoplastischen Materials bei Krafteinwirkung in einer Richtung gewonnen werden. Da mit Temperatureinwirkung auf thermoplastische Materialien ein Längsschrumpf LS und ein Querschrumpf QS des jeweiligen Materials einhergehen (vgl. Figur 2), sind die mittels der Vorrichtung 100 gemäß Figur 1 B gewonnen Werte häufig nicht ausreichend, um genau Vorhersagen zum jeweiligen Verhalten des zu schrumpfenden Materials im Schrumpfungsprozess treffen zu können.

Auch besitzen Vorrichtungen gemäß Ausführungsbeispiel der Figur 1B eine große Masse sowie hohe Anschaffungskosten. Auch aus diesem Grunde sind Messvorrichtungen zur Ermittlung von Schrumpfcharakteristika wünschenswert, welche die genannten Nachteile zumindest nicht vollständig aufweisen.

**Figur 2** zeigt beispielhaft den Längsschrumpf LS sowie den Querschrumpf QS mehrerer thermoplastischer Folien in Abhängigkeit der jeweiligen Schrumpfungstemperatur. So ist im obigen Koordinatensystem der Längsschrumpf LS mehrere thermoplastischer Folien dargestellte, wobei auf der Abszisse die Temperatur und auf der Ordinate der jeweilige Schrumpf des thermoplastischen Materials angetragen sind.

Wie sehr gut in Figur 2 zu erkennen, existieren thermoplastische Materialien, welche bei einer Temperatur von 120°C nahezu kein Schrumpfverhalten zeigen, während ein Schrumpfungsprozess bei weiteren thermoplastischen Materialien bereits ab einer Temperatur von ca. 110°C einsetzt. Den Längsschrumpf LS von thermoplastischen Materialien im jeweiligen Schrumpfungsprozess zu kennen, ist entscheidend, um eine Schrumpfung von thermoplastischen Folien auf Artikel optimiert ausbilden zu können.

Die Komplexität von Schrumpfungsprozessen wird weiter durch den in Figur 2 dargestellten Querschrumpf QS verdeutlicht. Auch hier ist auf der Abszisse die Temperatur und auf der Ordinate der Schrumpf für das jeweilige thermoplastische Material aufgetragen. Wird eine Temperatur von ca. 110°C überschritten, so zeigen diverse thermoplastische Materialien zunächst eine Ausdehnung bevor sie auf ein bestimmtes Niveau schrumpfen. Bei sämtlichen Materialien variiert der Querschrumpf QS in Abhängigkeit der Temperatur. Hierbei zeigen diverse Materialien über einen bestimmten Temperaturbereich eine zumindest näherungsweise lineare Zunahme der Schrumpfung, während bei weiteren Materialien keine lineare Zunahme der Schrumpfung zu verzeichnen ist. Auch hinsichtlich des Querschrumpfes QS von thermoplastischen Materialien besteht daher eine Notwendigkeit, das Verhalten bei Temperatureinwirkung bzw. des Verhalten in Abhängigkeit der jeweiligen Eigentemperatur möglichst genau zu kennen, um eine Schrumpfung von thermoplastischen Folien auf Artikel optimiert ausbilden zu können.

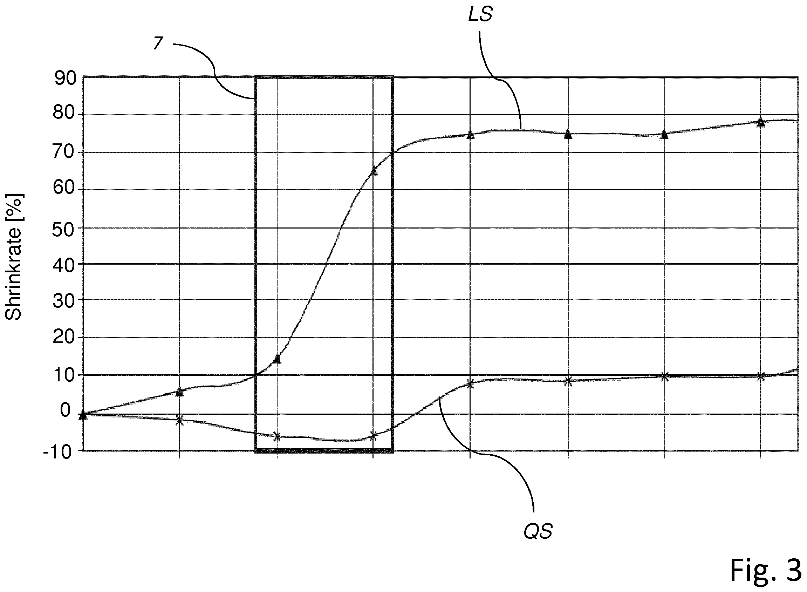

**Figur 3** zeigt beispielhaft den Längsschrumpf LS sowie den Querschrumpf QS einer thermoplastischen Folie aus der Praxis. Weiterhin ist auf der Abszisse die Temperatur des jeweiligen thermoplastischen Materials angetragen, während auf der Ordinate die prozentuale Schrumpfung des Materials festgesetzt ist. Bezugsziffer 7 verweist auf ein theoretisches Prozessfenster, innerhalb dessen in der Praxis Schrumpfungsprozesse von thermoplastischen Folien auf Artikel stattfinden.

Im dargestellten Bereich ist die Temperatur zu gering um einen Querschrumpf QS für das beispielhaft dargestellte thermoplastische Material auszubilden. Jedoch zeigt das Material im Temperaturbereich des theoretischen Prozessfensters 7 eine Ausdehnung des thermoplastischen Materials zwischen 0 und 10%.

Berücksichtigend den beispielhaft dargestellten Längsschrumpf LS ist bereits oberhalb einer Temperaturgrenze von 100°C eine Schrumpfung zu erkennen, welche im Prozessfenster 7 stark zunimmt und sich bei Verlassen des theoretischen Prozessfensters und ab einer Temperatur von ca. 120°C auf einen konstanten Wert zwischen 70% und 80% einpendelt.

Da der Längsschrumpf LS und der Querschrumpf QS, wie in Figur 3 zu erkennen, stark unterschiedlich ausgebildet sind, sind Vorrichtungen und Verfahren wünschenswert, vermittels welchen einen Längsschrumpf LS und ein Querschrumpf QS berücksichtigende Schrumpfungscharakteristika möglichst präzise feststellbar sind.

Da die in Figur 1B dargestellte Messvorrichtung 100 lediglich das Verhalten bei Krafteinwirkung auf das Probenstück 5 in einer Ausdehnungsrichtung berücksichtigt, sind über die Messvorrichtung 100 gewonnene Aussagen häufig nicht ausreichend, um genau vorhersagen über das Verhalten des jeweiligen thermoplastischen Materials bei Schrumpfung treffen zu können.

Zwar können ein Längsschrumpf LS sowie ein Querschrumpf QS gemäß Figur 3 mittels der aus dem Stand der Technik bekannten Vorrichtung 10 aus Figur 1A festgestellt werden, jedoch enthält eine Darstellung wie in Figur 3 keine Aussagen darüber, wie sich die Schrumpfung des jeweiligen thermoplastische Material bei Temperatureinwirkung über den Zeitverlauf verhält. Die Aussagen aus Figur 3, welche mittels einer Vorrichtung 10 gemäß Figur 1A gewonnen werden können, sind daher nicht ausreichend, um genaue Vorhersagen zum Verhalten einer thermoplastischen Folie bei Schrumpfung treffen zu können.

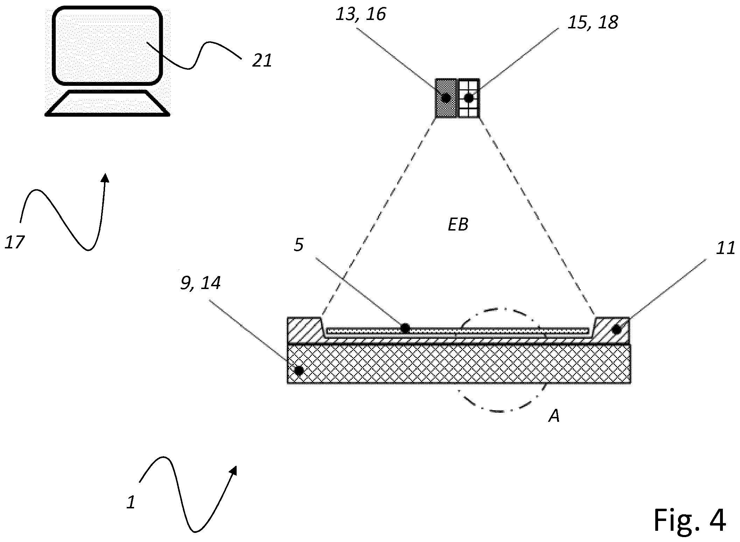

**Figur 4** zeigt eine schematische Ansicht eines Ausführungsbeispiels einer erfindungsgemäßen Vorrichtung 1 zur Feststellung von Schrumpfungscharakteristika einer flächigen Materialbahn aus thermoplastischem Kunststoff. Ein Probenstück 5 des jeweiligen thermoplastischen Kunststoffes ist bei Darstellung der Figur 4 bereits in die Aufnahme 11 der Vorrichtung 1 eingesetzt.

Die Aufnahme 11 der Vorrichtung 1 ist von außen zugänglich, so dass eine Person das jeweilige Probenstück 5 in die Aufnahme 11 der Vorrichtung 1 einsetzen bzw. einlegen kann. Zur Erwärmung des Probenstücks 5 steht die Aufnahme 11 mit einer Heizeinrichtung 9 in Wirkverbindung, welche vorliegend als elektrische Heizung 14 ausgebildet ist. Die Heizeinrichtung 9 bildet auf ihrer der Aufnahme 11 zugewandten Seite eine temperierbare Heizplatte aus, die mit der Aufnahme 11 zur Erwärmung des Probenstücks 5 flächig in Kontakt gebracht ist.

Über die Heizeinrichtung 9 ist die Aufnahme 11 derart temperierbar, dass das Probenstück 5 oberhalb einer für das jeweilige Probenstück 5 wirksamen Schrumpfungstemperatur erwärmt wird.

Weiter umfasst die Vorrichtung 1 eine Detektionseinrichtung 13. Über die Detektionseinrichtung 13 wird eine Dimensionsänderung des Probenstücks 5 nach Erwärmung oberhalb der jeweiligen Schrumpfungstemperatur über den Zeitverlauf beobachtet. Die Detektionseinrichtung 13 ist vorliegend als Kamerasystem 16 ausgebildet und steht mit einer rechnergestützten Einheit 17 in Verbindung, welche rechnergestützte Einheit 17 Daten zur sich über den Zeitverlauf ändernden Dimensionsänderung des Probenstücks 5 von der Detektionseinrichtung 13 bzw. dem Kamerasystem 16 empfängt.

Wie in Figur 2 zu erkennen, ist die Detektionseinrichtung 17 oberhalb der Aufnahme 11 bzw. oberhalb des Probenstücks 5 angeordnet und besitzt einen Erfassungsbereich EB, der sich über das gesamte in die Aufnahme 11 eingelegte Probenstück 5 erstreckt.

Zudem sind Mittel 15 zum Erfassen einer Temperatur und/oder Temperaturänderung des Probenstücks 5 dargestellt. Die Mittel 15 sind vorliegend ausgebildet durch einen oder mehrere Infrarotsensoren 18. Vorteilhafterweise ist durch die Ausbildung der Mittel 15 über Infrarotsensoren 18 kein direkter Kontakt zum Probenstück 5 notwendig, um die jeweilige Temperatur und/oder Temperaturänderung erfassen zu können. Die Dimensionsänderung des Probenstücks 5 wird somit bei Schrumpfung nicht verfälscht bzw. durch einen Kontakt von Sensoren mit dem Probenstück 5 nicht beeinflusst.

Auch stehen die Mittel 15 bzw. die Infrarotsensoren 18 mit der rechnergestützten Einheit 17 in Verbindung, wobei die rechnergestützte Einheit 17 bei Erfassen der Dimensionsänderung des Probenstücks 5 über den Zeitverlauf durchgehend Daten zur jeweiligen Dimensionsänderung von den Infrarotsensoren 18 empfängt.

Hierdurch kann die rechnergestützte Einheit 17 einen Zusammenhang aus einer Dauer des Schrumpfungsprozesses, der über die Mittel 15 erfassten Temperatur und/oder Temperaturänderung des Probenstücks 5 im Schrumpfungsprozess sowie der über den Zeitverlauf voranschreitenden Dimensionsänderung des Probenstücks 5 herstellen und ggf. als Schrumpfcharakteristika auf einer Anzeigeeinheit 21 darstellen. Weiter können die Schrumpfcharakteristika in vorstellbaren Ausführungsformen durch eine in Figur 2 nicht darstellte Druckeinrichtung ausgegeben werden, die ebenso mit der rechnergestützten Einheit 17 in Verbindung steht. Auch besitzt die rechnergestützte Einheit 17 eine Speichereinheit, auf welcher der jeweils hergestellte Zusammenhang bzw. die Schrumpfcharakteristika abgelegt werden. Vorhergehend kann eine Zuordnung der Schrumpfcharakteristika zu dem jeweiligen Probenstück 5 bzw. zu dem jeweiligen thermoplastischen Material des Probenstücks 5 hergestellt werden.

Um über die Schrumpfcharakteristika genau Aussagen zum Verhalten des Probenstücks 5 bzw. des thermoplastischen Materials des Probenstücks 5 über den Zeitverlauf treffen zu können, kann die Temperatur des Probenstücks 5 über die Mittel 15 bzw. die Infrarotsensoren 18 zumindest während der Dimensionsänderung des Probenstücks 5 durchgehend erfasst, an die rechnergestützte Einheit 21 übertragen und bei Herstellung des Zusammenhangs berücksichtigt werden.

Weiter kann vorgesehen sein, dass die Vorrichtung 1 einen Regelkreis ausbildet, bei welchem die Heizeinrichtung 9 über die rechnergestützte Einheit 21 mit den Mitteln 15 bzw. den Infrarotsensoren 18 in Wirkverbindung gebracht ist.

Beispielsweise ist denkbar, dass die Temperatur des Probenstücks 5 über den Zeitverlauf bei Erfassung der Dimensionsänderung des Probenstücks 5 zumindest weitgehend konstant gehalten werden soll. Wird eine zu geringe oder zu hohe Ist-Temperatur des Probenstücks 5 über die Mittel 15 erfasst, so kann über die rechnergestützte Einheit 17 die Heizeinrichtung 9 zur definierten Anpassung der Eigentemperatur des Probenstücks 5 angesteuert werden. Die Ansteuerung bzw. die Temperaturregelung kann hierbei zumindest näherungsweise in Echtzeit erfolgen.

In weiteren Ausführungsformen kann es sein, dass die jeweilige Temperatur bzw. das Temperaturniveau des Probenstücks 5 während der Dimensionsänderung definiert gehoben und gesenkt werden soll. Auch hierbei kann eine Erfassung der jeweiligen Ist-Temperatur des Probenstücks 5 über die Mittel 15 bzw. die Infrarotsensoren 18 erfolgen und die Heizeinrichtung 9 über die rechnergestützte Einheit 17 zur Anpassung der jeweiligen Temperatur des Probenstücks 5 angesteuert werden.

Ob vorteilhafterweise eine konstante Temperatur des Probenstücks 5 oder eine Temperaturänderung des Probenstücks 5 über den Zeitverlauf gewählt wird, kann in Abhängigkeit eines vorgesehen späteren Schrumpfungsprozesse, bei welchem das jeweilige thermoplastische Material auf Artikel aufgeschrumpft werden soll, wahlweise erfolgen.

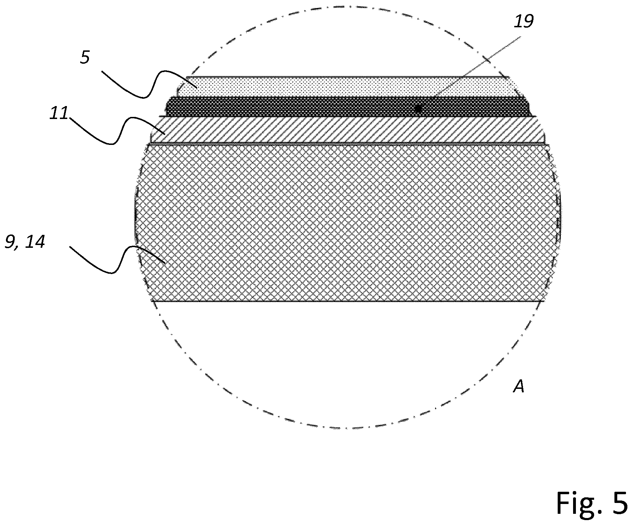

**Figur 5** zeigt eine vergrößerte Ansicht des Ausschnitts A aus Figur 4. Zu erkennen sind weiterhin die Heizeinrichtung 9, welche als elektrische Heizung 14 ausgebildet ist sowie die flächig mit der Heizeinrichtung 9 in Kontakt stehende Aufnahme 11. Zudem das Probenstück 5, welches in die Aufnahme 11 eingelegt ist.

Figur 5 zeigt weiter ein dem Probenstück 5 und der Aufnahme 11 zwischengeordnetes Trägersubstrat 19, welches vorliegend als Ölfilm ausgebildet ist, in weiteren Ausführungsformen jedoch durch alternative Trägermaterialien, wie beispielsweise Talkum, ausgebildet sein kann. Das Probenstück 5 ist hierbei mit einer seiner Breitseitenfläche auf das Trägersubstrat 19 aufgelegt und wird vom Trägersubstrat 19 derart getragen, dass es keinen direkten Kontakt bzw. keinen Oberflächenkontakt mit der Aufnahme 11 und der Heizeinrichtung 9 besitzt. Lediglich eine Breitseitenfläche des Probenstücks 5 ist hierbei mit dem Trägersubstrat 19 in Oberflächenkontakt stehend, während die weitere gegenüberliegende Breitseitenfläche des Probenstücks 5 keinen Oberflächenkontakt zum Trägersubstrat 19 besitzt und der Detektionseinrichtung 13 sowie den Infrarotsensoren 18 (vgl. Figur 4) zugewandt ist.

Da das Probenstück 5 mit einer seiner Breitseitenflächen vollständig mit dem Trägersubstrat 19 in Kontakt gebracht ist, kann via die Heizeinrichtung 9 thermische Energie an die Aufnahme 11 weitergegeben, von der Aufnahme 11 an das Trägersubstrat 19 übertragen und das Probenstück 5 über das Trägersubstrat 19 gleichmäßig erwärmt werden.

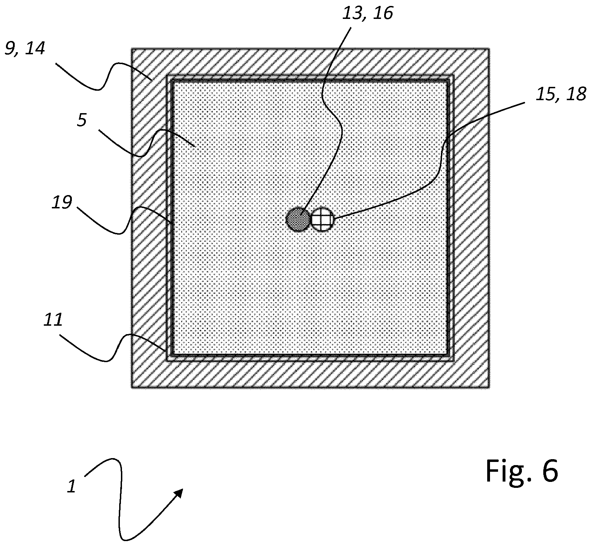

**Figur 6** zeigt eine schematische Draufsicht auf die Ausführungsform einer Vorrichtung 1 aus Figur 4. In Figur 6 sind weiterhin die Heizeinrichtung 9 zu erkennen, die Aufnahme 11, welche flächig mit einer Heizplatte der Heizeinrichtung 9 in Kontakt gebracht ist, das Trägersubstrat 19 sowie das in die Aufnahme 11 eingelegte Probenstück 5

Das Probenstück 5 besitzt vorliegend eine quadratische Geometrie und ist vor Erwärmung im Wesentlichen bündig in der Aufnahme 11 angeordnet. Die Geometrie des Probenstücks 5 in Figur 6 ist lediglich beispielhaft zu verstehen, so dass das Probenstück 5 in weiteren Ausführungsformen alternative Formgebungen besitzen kann.

Auch zeigt Figur 6 beispielhaft eine benachbarte Anordnung der Mittel 15 bzw. der ein oder mehreren Infrarotsensoren 18 sowie der Detektionseinrichtung 13 bzw. des Kamerasystems 16. Die Mittel 15 und die Detektionseinrichtung 13 sind oberhalb der Aufnahme 11 bzw. des in die Aufnahme 11 eingesetzten Probenstücks 5 angeordnet.

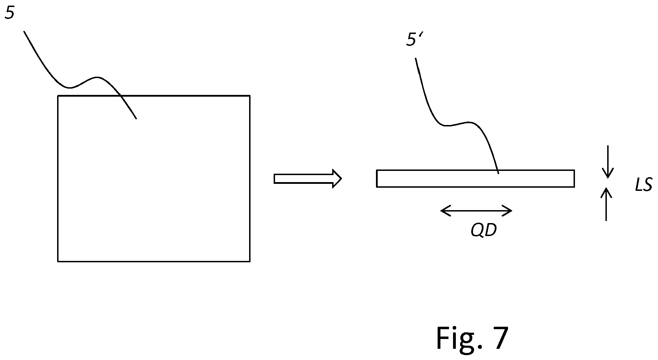

**Figur 7** zeigt eine beispielhafte Ausführungsform eines Probenstücks 5 vor Erwärmung sowie eine beispielhafte Ausführungsform eines Probenstücks 5' nach Erwärmung und erfolgter Schrumpfung.

Die Schrumpfung des Probenstücks 5 ist hierbei gemäß Diagramm aus Figur 3 in einem Temperaturbereich zwischen 110°C und 115°C erfolgt. Wie auch dem Diagramm zu entnehmen, ist im gewählten Temperaturbereich ein Längsschrumpf LS des Probenstücks 5 zu verzeichnen. Ein Querschrumpf QS findet im Temperaturbereich zwischen 110°C und 115°C nicht statt, jedoch erfolgt eine geringe Querdehnung QD zwischen 0% und 10% des Probenstücks 5. Mittels der erfindungsgemäßen Vorrichtung 1, wie sie beispielhaft in den Figuren 4 bis 6 dargestellte wurde, ist es möglich, sowohl den Längsschrumpf LS als auch die Querdehnung QD zu erfassen.

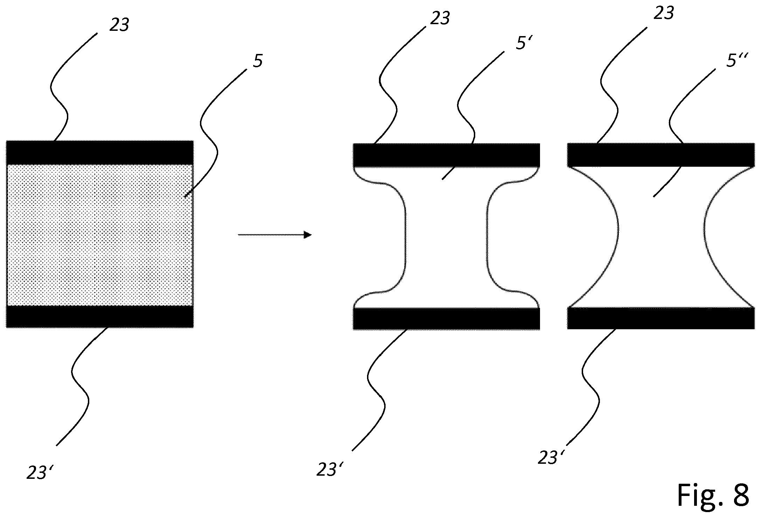

**Figur 8** zeigt eine schematische Ansicht einer weiteren beispielhaften Ausführungsform eines Probenstücks 5, wie es für diverse Ausführungsformen einer erfindungsgemäßen Vorrichtung 1 und zur Umsetzung bei diversen Ausführungsformen des erfindungsgemäßen Verfahrens Verwendung finden kann.

Das Probenstück 5 ist auf zwei gegenüberliegenden Rändern mit einem formstabilen Material 23 und 23' in Verbindung gebracht, welches formstabile Material 23 und 23' bei Erwärmung des Probenstücks 5 über die Heizeinrichtung 9 keine Dimensionsänderung erfährt. Vorliegend ist das formstabile Material 23 und 23' streifenförmig ausgebildet und auf das Probenstück 5 bzw. auf das thermoplastische Material des Probenstücks 5 aufgeklebt.

Zwei mögliche Dimensionsänderungen des Probenstücks 5, wie sie in Abhängigkeit des jeweiligen für das Probenstück 5 gewählten thermoplastischen Materials vorliegen können sind in Figur 8 rechtsseitig dargestellt.

So zeigt das erste rechtsseitig dargestellte Probenstück 5' eine Dimensionsänderung, bei welcher ein Schrumpfungsprozess des Probenstücks 5' im Bereich der formstabilen Materialien 23 und 23' gering ausgebildet ist und ab einer gewissen Entfernung von den formstabilen Materialien 23 und 23' jeweils stark zunimmt. Ab einer gewissen Entfernung von den formstabilen Materialien 23 und 23' ist die Schrumpfung des Probenstücks 5' konstant ausgebildet.

Das weitere Probenstück 5" zeigt eine Dimensionsänderung, bei welcher die Schrumpfung des Probenstücks 5" in Richtung weg von den formstabilen Materialien 23 und 23' kurvenförmig verläuft. Gegenüber dem Probenstück 5' ist die Schrumpfung im Bereich der formstabilen Materialien 23 und 23' bei Probenstück 5" verstärkt ausgebildet.

Mittels einer Ausführungsform eines Probenstücks 5 bzw. 5' und 5" gemäß Figur 5 sowie formstabilen Materialien 23 und 23' lassen sich weitere Aussagen zum Verhaltens des jeweiligen thermoplastischen Materials bei Aufschrumpfung auf Artikel treffen.

Da die formstabilen Materialien 23 und 23', ebenso wie Artikel bei Aufschrumpfung des thermoplastischen Materials, einen Widerstand ausbilden, kann ein Schrumpfungsprozess, bei welchen thermoplastisches Material auf Artikel aufgeschrumpft wird, simuliert werden. Die eingenommene Geometrie bzw. die Dimensionsänderung der jeweiligen Probenstücke 5' und 5" erlaubt die Feststellung weiterer und mittels Vorrichtungen gemäß den Figuren 1A und 1B nicht feststellbarer Schrumpfungscharakteristika.

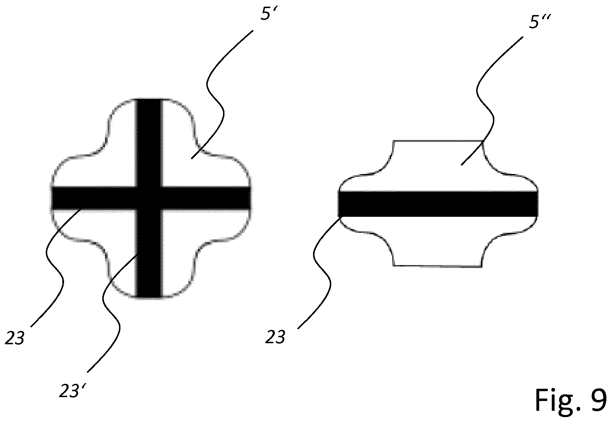

**Figur 9** zeigt eine schematische Ansicht einer weiteren beispielhaften Ausführungsform zweier Probenstücke 5' und 5" nach einem Schrumpfungsprozess.

Ebenso wie die Probenstücke 5' bzw. 5" aus dem Ausführungsbeispiel der Figur 8 sind auf die Probenstücke 5' und 5" aus Figur 9 formstabile Materialien 23 bzw. 23' aufgeklebt. So zeigt das linksseitig dargestellte Probenstück 5' lotrecht zueinander orientierte formstabile Materialien 23 und 23', die jeweils eine Symmetrieachse für das Probenstück 5' ausbilden. Die formstabilen Materialien 23 und 23' des linksseitig dargestellten Probenstücks 5' schneiden sich hierbei und sind überlappend auf das Probenstück 5' aufgeklebt.

Weiter umfasst das rechtsseitig dargestellte Probenstück 5" lediglich einen Streifen an formstabilem Material 23, der ebenso als Symmetrieachse für das Probenstück 5" ausgebildet ist.

Die Ausführungsbeispiele aus den Figuren 8 und 9 dienen lediglich zur Verdeutlichung, wie ggf. formstabiles Material 23 bzw. 23' auf Probenstücke 5 bzw. 5' und/oder 5" aufgebracht werden kann. Für den angesprochenen Fachmann ist klar, dass er in diversen Ausführungsformen für Probenstücke 5 bzw. 5' und/oder 5" alternative Anordnungen von formstabilen Materialien 23 und/oder 23' vorsehen kann, um mittels der erfindungsgemäßen Vorrichtung 1 bzw. mittels dem erfindungsgemäßen Verfahren Aussagen zum Verhalten des jeweiligen thermoplastischen Materials bei Aufschrumpfung auf Artikel treffen zu können.

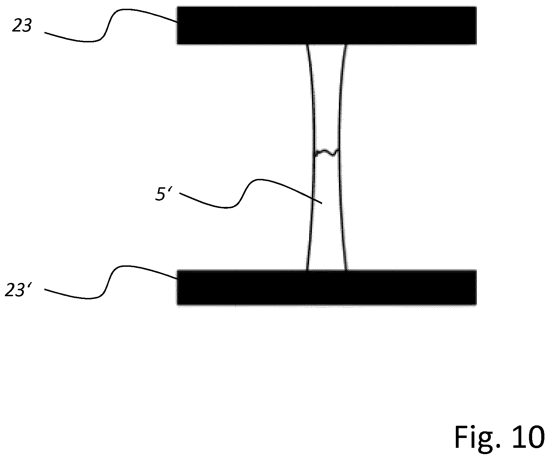

**Figur 10** zeigt eine schematische Ansicht eines Probenstücks 5' bei Umsetzung von möglichen Ausführungsformen eines erfindungsgemäßen Verfahrens; Das Probenstück 5' aus Figur 10 wurde bereits oberhalb einer für das Probenstück 5' wirksamen Schrumpfungstemperatur erwärmt.

Ebenso wie die Probenstücke 5 bzw. 5' und 5" aus dem Ausführungsbeispiel der Figur 8 wurden auf das Probenstück 5' aus dem Ausführungsbeispiel der Figur 10 Steifen von formstabilen Materialien 23 und 23' aufgeklebt.

Die formstabilen Materialien 23 und 23' sind jedoch ortsfest in der Aufnahme 11 (vgl. Figuren 4 bis 6) angeordnet und können sich bei Schrumpfung des Probenstücks 5' nicht zusammen mit dem Probenstück 5' bewegen. Da sich das Probenstück 5' bei Erwärmung oberhalb seiner wirksamen Schrumpfungstemperatur im Längsschrumpf LS zusammenzieht, wurde das Probenstück 5' aufgrund des Schrumpfprozesses bis zum Bruch gedehnt. Unter Berücksichtigung der Dimensionsänderung bei Verbindung des Probenstücks 5' mit ortsfest in der Aufnahme 11 angeordneten formstabilen Materialien sowie dem einsetzenden Bruch bei Überdehnung lassen sich weitere Aussagen gewinnen, welche als Schrumpfcharakteristika präzise Vorhersagen zum Verhalten des thermoplastischen Materials bei Aufschrumpfung auf Artikel erlauben.

Die Erfindung wurde unter Bezugnahme auf eine bevorzugte Ausführungsform beschrieben. Es ist jedoch für einen Fachmann vorstellbar, dass Abwandlungen oder Änderungen der Erfindung gemacht werden können, ohne dabei den Schutzbereich der nachstehenden Ansprüche zu verlassen.

### Bezugszeichenliste

- 1: Vorrichtung
- 5: Probenstück
- 7: Theoretisches Prozessfenster
- 9: Heizeinrichtung
- 10: Messvorrichtung
- 11: Aufnahme
- 13: Detektionseinrichtung
- 14: Elektrische Heizung
- 15: Mittels zur Temperaturerfassung
- 16: Kamerasystem
- 17: Rechnergestützte Einheit
- 18: Infrarotsensoren
- 19: Trägersubstrat
- 21: Anzeigeeinheit
- 23: Formstabiles Material
- 30: Flüssigkeitsbad
- 50: Temperaturregler
- 100: Messvorrichtung
- 110: Unterer Spannkopf
- 120: Oberer Spannkopf
- 150: Maschinenrahmen
- 170: Arbeitskolben

- LS: Längsschrumpf
- QD: Querdehnung
- QS: Querschrumpf

## Patentansprüche

1. Verfahren zur Feststellung von Schrumpfungscharakteristika einer flächigen Materialbahn aus thermoplastischem Kunststoff, bei dem ein Probenstück (5) definierter Abmessung einer Erwärmung oberhalb einer für das jeweilige Material des Probenstücks (5) wirksamen Schrumpfungstemperatur unterzogen wird, wobei nach Erwärmung über den Zeitverlauf eine Dimensionsänderung des Probenstücks (5) im voranschreitenden Schrumpfungsprozess beobachtet wird und ein Zusammenhang zur Dauer des Schrumpfungsprozesses sowie zur jeweiligen Temperatur und/oder Temperaturänderung des Probenstücks (5) im Schrumpfungsprozess hergestellt wird, **dadurch gekennzeichnet, dass** die Dimensionsänderung des Probenstücks (5) im voranschreitenden Schrumpfungsprozess vermittels optischer Erkennung und hierbei über ein oder mehrere Kamerasysteme (16) beobachtet wird.

2. Verfahren nach Anspruch 1, bei welchem die jeweilige Temperatur und/oder Temperaturänderung des Probenstücks (5) zumindest während dem Beobachten und/oder Messen der Dimensionsänderung durchgehend erfasst werden.

3. Verfahren nach Anspruch 1 oder Anspruchs 2, bei welchem die Temperatur und/oder die Temperaturänderung des Probenstücks (5) vermittels eines oder mehrerer Infrarotsensoren (18) erfasst werden.

4. Verfahren nach einem oder mehreren der Ansprüche 1 bis 3, bei welchem das Probenstück (5) auf ein Trägersubstrat (19) aufgelegt wird, welches zur Erwärmung thermische Energie an das jeweilige Probenstück (5) weitergibt.

5. Verfahren nach einem oder mehreren der Ansprüche 1 bis 4, bei welchem das Probenstück (5) vor Erwärmung bereichsweise mit einem formstabilen Material (23, 23') flächig in Verbindung gebracht wird, welches formstabile Material (23, 23') derart ausgebildet ist, dass es bei Erwärmung des Probenstücks (5) keine oder im Wesentlichen keine Dimensionsänderung erfährt.

6. Verfahren nach Anspruch 5, bei welchem zwei Einheiten aus formstabilem Material (23, 23') auf unterschiedliche Bereiche des Probenstücks (5) aufgebracht sind, wobei die zwei Einheiten bei Dimensionsänderung des Probenstücks (5) ortsfest gehalten werden.

7. Verfahren nach Anspruch 5 oder Anspruch 6, bei welchem ein erstes Probenstück (5) des thermoplastischen Kunststoffes vor Erwärmung bereichsweise mit dem formstabilen Material (23, 23') in Verbindung gebracht wird und ein zweites Probenstück (5) des thermoplastischen Kunststoffes nicht mit dem formstabilen Material (23, 23') in Verbindung gebracht wird, wobei das erste und das zweite Probenstück (5) zeitgleich erwärmt werden und ein Zusammenhang unter Berücksichtigung der Dimensionsänderungen des ersten und des zweiten Probenstücks (5) im voranschreitenden Schrumpfungsprozess, der jeweiligen Temperatur und/oder Temperaturänderung des ersten und des zweiten Probenstücks (5) im Schrumpfungsprozess sowie der Dauer des Schrumpfungsprozesses hergestellt wird.

8. Verfahren nach Anspruch 1, bei welchem zur optischen Erkennung der Dimensionsänderung wenigstens eine visuelle Kennzeichnung auf das jeweilige Probenstück (5) aufgebracht wird.

9. Vorrichtung (1) zur Feststellung von Schrumpfungscharakteristika einer flächigen Materialbahn aus thermoplastischem Kunststoff, umfassend
- eine Aufnahme (11) für ein Probenstück (5) des jeweiligen thermoplastischen Kunststoffes,
- eine Temperiereinrichtung (9), welche mit der Aufnahme (11) zur Erwärmung des Probenstücks (5) oberhalb einer für das jeweilige Material wirksamen Schrumpfungstemperatur in Verbindung steht,
- wenigstens eine Detektionseinrichtung (13) zum Beobachten einer voranschreitenden Dimensionsänderung des Probenstücks (5) über den Zeitverlauf während eines aus der Erwärmung resultierenden Schrumpfungsprozesses,
- ein oder mehrere Mittel (15) zum Erfassen einer Temperatur und/oder Temperaturänderung des Probenstücks (5) im Schrumpfungsprozess und
- eine rechnergestützte Einheit (17), wobei
die rechnergestützte Einheit (17) mit den ein oder mehreren Mitteln (15) zum Erfassen einer Temperatur und/oder Temperaturänderung des Probenstücks (5) und mit der wenigstens einen Detektionseinrichtung (13) zum Beobachten einer Dimensionsänderung des jeweiligen Probenstücks (5) derart in Wirkverbindung gebracht ist, dass über die rechnergestützte Einheit (17) ein Zusammenhang aus einer Dauer des Schrumpfungsprozesses, der erfassten Temperatur und/oder Temperaturänderung des Probenstücks (5) im Schrumpfungsprozess sowie der über den Zeitverlauf voranschreitenden Dimensionsänderung des Probenstücks (5) herstellbar ist, **dadurch gekennzeichnet, dass** die wenigstens eine Detektionseinrichtung (13) zum Erfassen einer Dimensionsänderung des Probenstücks (5) zumindest ein Kamerasystem (16) umfasst.

10. Vorrichtung (1) nach Anspruch 9, bei welcher über die rechnergestützte Einheit (17) aus einer durchgehend während dem Messen und/oder Beobachten der voranschreitenden Dimensionsänderung ausgebildeten Erfassung einer Temperatur und/oder Temperaturänderung des Probenstücks (5) ein Zusammenhang herstellbar ist.

11. Vorrichtung (1) nach Anspruch 9 oder Anspruch 10, bei welcher die ein oder mehreren Mittel (15) zum Erfassen einer Temperatur und/oder Temperaturänderung des Probenstücks (5) wenigstens einen Infrarotsensor (18) umfassen.

12. Vorrichtung (1) nach einem oder mehreren der Ansprüche 9 bis 11, bei welcher das Probenstück (5) in der Aufnahme (11) auf ein Trägersubstrat (19) auflegbar ist, welches zur Weitergabe von thermischer Energie an das jeweilige Probenstück (5) mit einer Heizplatte der Temperiereinrichtung (9) in Verbindung steht.

13. Vorrichtung (1) nach Anspruch 12, bei welcher das Trägersubstrat (19) durch Öl und/oder Talkum ausgebildet ist.

14. Vorrichtung (1) nach einem oder mehreren der Ansprüche 9 bis 13, bei welcher die Temperiereinrichtung durch eine elektrisch- und/oder gasbetriebene Heizung (14) ausgebildet ist.

15. Vorrichtung (1) nach einem oder mehreren der Ansprüche 9 bis 14, bei welcher die rechnergestützte Einheit (17) zur Weitergabe von Informationen über den hergestellten Zusammenhang mit einer Steuerungseinrichtung eines Schrumpftunnels in Verbindung steht.

## Claims

1. A method for determining shrinking features of a planar material web of thermoplastic material, in which a test piece (5) of specified dimensions undergoes a heat treatment above a shrinking temperature that is effective for the particular material of the test piece (5), wherein a dimensional change of the test piece (5) over time in the proceeding shrinking process is observed after the heat treatment, and a connection is established to the duration of the shrinking process as well as to the particular temperature and/or temperature change of the test piece (5) in the shrinking process, **characterised in that** the dimensional change of the test piece (5) in the proceeding shrinking process is observed by means of optical identification and in this context by one or more camera systems (16).

2. The method according to claim 1, in which the particular temperature and/or temperature change of the test piece (5) are detected continuously at least during the observation and/or measurement of the dimensional change.

3. The method according to claim 1 or claim 2, in which the temperature and/or the temperature change of the test piece (5) are detected by means of one or more infrared sensors (18).

4. The method according to one or more of the claims 1 to 3, in which the test piece (5) is applied onto a carrier substrate (19) that transfers thermal energy for heat treatment to the particular test piece (5).

5. The method according to one or more of the claims 1 to 4, in which the test piece (5) is in some sections brought into planar contact with a dimensionally stable material (23, 23') prior to heat treatment, with the dimensionally stable material (23, 23') being designed such that it undergoes no or essentially no dimensional change during heat treatment of the test piece (5).

6. The method according to claim 5, in which two units of dimensionally stable material (23, 23') are applied onto different areas of the test piece (5), wherein the two units are fixed in place during dimensional change of the test piece (5).

7. The method according to claim 5 or claim 6, in which a first test piece (5) of the thermoplastic material is in some sections brought into contact with the dimensionally stable material (23, 23') prior to heat treatment, and a second test piece (5) of the thermoplastic material is not brought into contact with the dimensionally stable material (23, 23'), wherein the first and the second test pieces (5) are simultaneously heat-treated, and a connection is established in consideration of the dimensional changes of the first and the second test pieces (5) in the proceeding shrinking process, of the particular temperature and/or temperature change of the first and the second test pieces (5) in the shrinking process, as well as of the duration of the shrinking process.

8. The method according to claim 1, in which at least one visual marking is applied onto the particular test piece (5) for the purpose of optical identification of the dimensional change.

9. A device (1) for determining shrinking features of a planar material web of thermoplastic material, the device (1) comprising
- a reception (11) for a test piece (5) of the particular thermoplastic material;
- a temperature control device (9) being linked to the reception (11) for the purpose of the heat treatment of the test piece (5) above a shrinking temperature that is effective for the particular material;
- at least one detection device (13) for observing a proceeding dimensional change of the test piece (5) over time during a shrinking process resulting from the heat treatment;
- one or more means (15) for detecting a temperature and/or a temperature change of the test piece (5) in the shrinking process; and
- a computer-based unit (17), wherein
the computer-based unit (17) is brought into operative connection with the one or more means (15) for detecting a temperature and/or a temperature change of the test piece (5) and with the at least one detection device (13) for observing a dimensional change of the particular test piece (5) such that a connection is establishable by way of the computer-based unit (17) from the duration of the shrinking process, from the detected temperature and/or temperature change of the test piece (5) in the shrinking process, as well as from the dimensional change of the test piece (5) proceeding over time, **characterised in that** the at least one detection device (13) for detecting a dimensional change of the test piece (5) comprises at least one camera system (16).

10. The device (1) according to claim 9, in which a connection is establishable by way of the computer-based unit (17) from a detection of a temperature and/or of a temperature change of the test piece (5), the detection being formed to be continuous during the measurement and/or observation of the proceeding dimensional change.

11. The device (1) according to claim 9 or claim 10, in which the one or more means (15) for detecting a temperature and/or a temperature change of the test piece (5) comprise at least one infrared sensor (18).

12. A device (1) according to one or more of the claims 9 to 11, in which the test piece (5) in the reception (11) is applicable onto a carrier substrate (19) being linked to a heating plate of the temperature control device (9) for the purpose of transferring thermal energy to the particular test piece (5).

13. A device (1) according to claim 12, in which the carrier substrate (19) is formed by oil and/or talcum.

14. The device (1) according to one or more of the claims 9 to 13, in which the temperature control device is formed by an electrically powered heater (14) and/or by a gas-powered heater (14).

15. The device (1) according to one or more of the claims 9 to 14, in which the computer-based unit (17) is linked to a control device of a shrink tunnel for the purpose of transferring information on the established connection.

## Revendications

1. Procédé de détermination de caractéristiques de rétraction d'une bande plane de matière en matière thermoplastique, dans lequel un échantillon (5) de dimension définie est soumis à un chauffage au-dessus d'une température de rétraction efficace pour la matière respective de l'échantillon (5), dans lequel, après le chauffage, on observe au cours du temps un changement dimensionnel de l'échantillon (5) dans le processus de rétraction progressif, et on établit une relation avec la durée du processus de rétraction ainsi qu'avec la température et/ou variation de température respective de l'échantillon (5) dans le processus de rétraction, **caractérisé par le fait que** le changement dimensionnel de l'échantillon (5) dans le processus de rétraction progressif est observé par l'intermédiaire de détection optique et ici au moyen d'un ou de plusieurs systèmes de caméra (16).

2. Procédé selon la revendication 1, dans lequel la température et/ou variation de température respective de l'échantillon (5) sont détectées de façon continue au moins durant l'observation et/ou la mesure du changement dimensionnel.

3. Procédé selon la revendication 1 ou la revendication 2, dans lequel la température et/ou la variation de température de l'échantillon (5) sont détectées par le biais d'un ou de plusieurs capteurs infrarouges (18).

4. Procédé selon l'une ou plusieurs des revendications 1 à 3, dans lequel ledit échantillon (5) est posé sur un substrat porteur (19) qui transmet de l'énergie thermique à l'échantillon (5) respectif pour le chauffage.

5. Procédé selon l'une ou plusieurs des revendications 1 à 4, dans lequel, avant le chauffage, l'échantillon (5) est mis en contact en nappe par zones avec une matière de forme stable (23, 23'), laquelle matière de forme stable (23, 23') est conçue de manière à ce qu'elle ne subisse pas ou ne subisse sensiblement pas de changement dimensionnel lorsque l'échantillon (5) est chauffé.

6. Procédé selon la revendication 5, dans lequel deux unités en matière de forme stable (23, 23') sont appliquées sur des zones différentes de l'échantillon (5), les deux unités étant maintenues à poste fixe lors d'un changement dimensionnel de l'échantillon (5).

7. Procédé selon la revendication 5 ou la revendication 6, dans lequel un premier échantillon (5) de la matière thermoplastique est mis en contact par zones, avant le chauffage, avec la matière de forme stable (23, 23'), et un deuxième échantillon (5) de la matière thermoplastique n'est pas mis en contact avec la matière de forme stable (23, 23'), dans lequel les premier et deuxième échantillons (5) sont chauffés en même temps et une relation est établie en prenant en considération les changements dimensionnels des premier et deuxième échantillons (5) dans le processus de rétraction progressif, la température et/ou variation de température respective des premier et deuxième échantillons (5) dans le processus de rétraction ainsi que la durée du processus de rétraction.

8. Procédé selon la revendication 1, dans lequel, pour détecter optiquement le changement dimensionnel, au moins un repère visuel est réalisé sur l'échantillon (5) respectif.

9. Dispositif (1) de détermination de caractéristiques de rétraction d'une bande plane de matière en matière thermoplastique, comprenant
- un logement (11) pour un échantillon (5) de la matière thermoplastique respective,
- un dispositif à tempérer (9) qui est en liaison avec le logement (11) pour le chauffage de l'échantillon (5) au-dessus d'une température de rétraction efficace pour la matière respective,
- au moins un dispositif de détection (13) pour observer un changement dimensionnel progressif de l'échantillon (5) au cours du temps durant un processus de rétraction résultant du chauffage,
- un ou plusieurs moyen(s) (15) de détection d'une température et/ou d'une variation de température de l'échantillon (5) dans le processus de rétraction et
- une unité assistée par ordinateur (17), dans lequel
ladite unité assistée par ordinateur (17) est mise en liaison active avec ledit un ou lesdits plusieurs moyen(s) (15) de détection d'une température et/ou d'une variation de température de l'échantillon (5) et avec ledit au moins un dispositif de détection (13) pour observer un changement dimensionnel de l'échantillon (5) respectif, de telle sorte qu'une relation puisse être établie par l'intermédiaire de l'unité assistée par ordinateur (17) à partir d'une durée du processus de rétraction, de la température et/ou variation de température détectée(s) de l'échantillon (5) dans le processus de rétraction ainsi que du changement dimensionnel progressif dans le temps de l'échantillon (5), **caractérisé par le fait que** ledit au moins un dispositif de détection (13) pour détecter un changement dimensionnel de l'échantillon (5) comprend au moins un système de caméra (16).

10. Dispositif (1) selon la revendication 9, dans lequel une relation peut être établie par l'intermédiaire de l'unité assistée par ordinateur (17) à partir d'une détection d'une température et/ou variation de température de l'échantillon (5), qui est réalisée de façon continue durant la mesure et/ou l'observation du changement dimensionnel progressif.

11. Dispositif (1) selon la revendication 9 ou la revendication 10, dans lequel ledit un ou ladite pluralité de moyens (15) de détection d'une température et/ou variation de température de l'échantillon (5) comprend au moins un capteur infrarouge (18).

12. Dispositif (1) selon l'une ou plusieurs des revendications 9 à 11, dans lequel l'échantillon (5) peut être posé sur un substrat porteur (19) à l'intérieur du logement (11), qui, pour transmettre de l'énergie thermique à l'échantillon (5) respectif, est en liaison avec une plaque chauffante du dispositif à tempérer (9).

13. Dispositif (1) selon la revendication 12, dans lequel ledit substrat porteur (19) est formé par de l'huile et/ou du talc.

14. Dispositif (1) selon l'une ou plusieurs des revendications 9 à 13, dans lequel le dispositif à tempérer est formé par un chauffage (14) fonctionnant de manière électrique et/ou au gaz.

15. Dispositif (1) selon l'une ou plusieurs des revendications 9 à 14, dans lequel, pour transmettre des informations sur la relation établie, ladite unité assistée par ordinateur (17) est en liaison avec un dispositif de commande d'un tunnel de rétraction.
